(19) Europäisches Patentamt
European Patent Office
Office européen des brevets

(11) EP 2 766 332 B1

(12) # FASCICULE DE BREVET EUROPEEN

(45) Date de publication et mention de la délivrance du brevet:
**18.04.2018 Bulletin 2018/16**

(21) Numéro de dépôt: **12780221.3**

(22) Date de dépôt: **03.10.2012**

(51) Int Cl.:
***C07C 29/04*** *(2006.01)* ***C07C 31/10*** *(2006.01)*
***C12P 7/04*** *(2006.01)* ***C12R 1/01*** *(2006.01)*
***C12R 1/145*** *(2006.01)* ***C12R 1/19*** *(2006.01)*

(86) Numéro de dépôt international:
**PCT/FR2012/052239**

(87) Numéro de publication internationale:
**WO 2013/054022 (18.04.2013 Gazette 2013/16)**

(54) **PRODUCTION D'ISOPROPANOL PAR DES SOUCHES RECOMBINANTES AMELIOREES**

HERSTELLUNG VON ISOPROPANOL DURCH VERBESSERTE REKOMBINANTE STÄMME

PRODUCTION OF ISOPROPANOL BY IMPROVED RECOMBINANT STRAINS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.10.2011 FR 1159175**

(43) Date de publication de la demande:
**20.08.2014 Bulletin 2014/34**

(73) Titulaires:
- **IFP Energies nouvelles**
  **92500 Rueil-Malmaison (FR)**
- **Stichting Dienst Landbouwkundig Onderzoek**
  **6701 BH Wageningen (NL)**

(72) Inventeurs:
- **COLLAS, Florent**
  **6701AM Wageningen (NL)**
- **MARCHAL, Rémy**
  **78400 Chatou (FR)**
- **CLEMENT, Benjamin**
  **94100 Saint Maur des Fosses (FR)**
- **LOPEZ CONTRERAS, Ana Maria**
  **3522GL Utrecht (NL)**
- **CLAASSEN, Pieternel A. M.**
  **6704PM Wageningen (NL)**

(74) Mandataire: **Bourgouin, André et al**
**Grosset-Fournier & Demachy**
**54, rue Saint Lazare**
**75009 Paris (FR)**

(56) Documents cités:
**EP-A1- 2 182 051** **WO-A1-2008/131286**
**WO-A1-2009/103026**

- **HANAI T ET AL: "Engineered synthetic pathway for isopropanol production in Escherichia coli", APPLIED AND ENVIRONMENTAL MICROBIOLOGY DECEMBER 2007 AMERICAN SOCIETY FOR MICROBIOLOGY US, vol. 73, no. 24, décembre 2007 (2007-12), pages 7814-7818, XP002671906, DOI: DOI:10.1128/AEM.01140-07 cité dans la demande**
- **TORU JOJIMA ET AL: "Production of isopropanol by metabolically engineered Escherichia coli", APPLIED MICROBIOLOGY AND BIOTECHNOLOGY, SPRINGER, BERLIN, DE, vol. 77, no. 6, 7 novembre 2007 (2007-11-07), pages 1219-1224, XP019586231, ISSN: 1432-0614, DOI: 10.1007/S00253-007-1246-8 [extrait le 2007-11-07] cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 766 332 B1

## Description

La présente invention concerne un procédé de production d'isopropanol par des souches recombinantes améliorées.

## Étude bibliographique

L'isopropanol, appelé également 2-propanol, ou alcool isopropylique, est utilisé principalement comme solvant pour la préparation des encres et des agents tensio-actifs. Ses autres applications concernent les propriétés d'antiseptique, de solvant de la carboxymethylcellulose (CMC). L'isopropanol est également utilisé dans la production de bases cosmétologiques, comme solvant des pesticides ou en tant que source de matière en synthèse organique. A titre indicatif, on peut citer que la production d'isopropanol s'élevait à 45 millions de tonnes aux USA en 1990 (Papa A, 2005).

De nos jours, l'isopropanol est produit par une méthode essentiellement chimique consistant en l'hydratation du propylène selon une voie directe ou une voie indirecte. La voie indirecte met en oeuvre deux étapes. Dans la première étape, on produit un mélange d'esters sulfuriques mono- et di-isopropyliques, qui sont hydrolysés en isopropanol dans une seconde étape. La voie directe met en oeuvre l'hydratation du propylène à haute pression et basse température sur un catalyseur "lit fixe" acide (Logsdon J.E. and Loke R. A, 2001).

De 1942 à 1958, l'isopropanol a été produit par voie fermentaire à Taiwan (Rogers et al. 2006). Les substrats de la fermentation étaient la patate douce, le manioc, l'amidon de blé, tandis que le microorganisme était une souche sauvage. De nos jours, ce procédé de production d'isopropanol n'est plus utilisé industriellement. En effet les faibles productions obtenues avec les souches sauvages (entre 2 et 3 g/L d'isopropanol pour *Clostridium. beijerinckii*) ne permettent pas la mise en place de procédés économiquement viables. Tout comme la fermentation solvantogène ABE (Acétone, Butanol, Éthanol), la fermentation IBE (Isopropanol, Butanol, Éthanol) a été abandonnée au début des années 1960 parallèlement au développement de l'industrie pétrochimique.

En raison de soucis écologiques et économiques, la recherche sur la production d'isopropanol par fermentation microbiologique a suscité un regain d'intérêt ces dernières années. La fermentation productrice d'isopropanol est connue sous le nom de fermentation IBE. Elle se distingue d'une autre fermentation productrice de solvants, la fermentation ABE, en ce qu'elle produit de l'isopropanol, uniquement ou en majeure partie, aux dépens de l'acétone. Les souches productrices de solvants appartiennent au genre *Clostridium.* Elles ont été reclassées en quatre espèces : *C. acetobutylicum*, *C. beijerinckii*, *C. aurantibutyricum*, *C. saccharoperbutylacetonicum* (Keis et al. 2001).

*C. acetobutylicum* est une souche utilisée fréquemment dans la fermentation ABE. Les produits principaux de fermentation avec C. *acetobutylicum* sont donc l'acétone, l'éthanol et le butanol. L'isopropanol n'est pas produit par les souches sauvages de C. *acetobutylicum.* L'isopropanol est un produit caractéristique d'un micro-organisme anciennement dénommé *"Clostridium butylicum"* (Osburn et al. 1937 ; Langlykke et al. 1937). En 1983, George *et al.* ont montré que C. *butylicum* était similaire à C. *beijerinckii.* Dans la classification actuelle, l'espèce C. *beijerinckii* contient donc des souches qui étaient anciennement classées en tant que *C. butylicum.* Cependant, toutes les souches de C. *beijerinckii* ne produisent pas de l'isopropanol. Par exemple, la souche C. *beijerinckii* NRRL B593 en produit, alors que la souche C. *beijerinckii* NRRL B592 n'en produit pas.

La voie de biosynthèse de l'isopropanol chez C. *beijerinckii* comporte les étapes suivantes :

(i) deux molécules d'acétyl-CoA sont condensées en une molécule d'acétoacétyl-CoA par l'acétyl-CoA transférase (EC 2.3.1.9), nommée également la thiolase (Thl) ;

(ii) le CoA de l'acétylCoA est transféré à l'acétate ou au butyrate par l'acétoacétyl-CoA transférase (Ca_CtfAB) (EC 2.8.3.9) pour donner l'acétoacétate ;

(iii) l'acétoacétate est ensuite converti en acétone et $CO_2$ par l'acétate décarboxylase (Ca_Adc) (EC 4.1.1.4) ;

(iv) enfin, l'acétone est converti en isopropanol par l'alcool déshydrogénase secondaire (Cb_s-Adh) (EC 1.1.1.2).

La comparaison des activités enzymatiques chez C. *beijerinckii* NRRL B593 et C. *beijerinckii* NRRL B592 (qui, respectivement, produit et ne produit pas d'isopropanol) a montré que la capacité d'excrétion de l'isopropanol était liée à l'activité alcool déshydrogénase secondaire Cb_s-Adh (Yan et al. 1988). L'enzyme responsable a été purifiée à partir d'extraits acellulaires de C. *beijerinckii* NRRL B593 (Ismaiel et al. 1993). La déshydrogénase de C. *beijerinckii* NEST255 a également été purifiée à des fins de comparaison. Les alcools déshydrogénases secondaires des souches C. *beijerinckii* NRRL B593 et C. *beijerinckii* NEST255 sont dépendantes du NADP. *In vitro*, elles utilisent comme attendu les alcools secondaires (isopropanol), mais aussi les alcools primaires, notamment le butanol, avec une activité cependant plus faible. L'étude cinétique de l'enzyme a confirmé que le substrat physiologique de l'alcool déshydrogénase secondaire était bien l'acétone et non l'isopropanol (Chen et al. 1995). L'alcool déshydrogénase secondaire de C. *beijerinckii* (Cb-s-Adh) est clairement distincte de l'alcool déshydrogénase primaire issue du même micro-organisme, qui réduit, *in vivo*, le butyraldéhyde en butanol.

Le gène codant pour Cb_s-Adh a été séquencé (Petretz et al. 1997). La structure de l'enzyme a été déterminée par les rayons X. Cb_s-Adh est une enzyme homotetramérique à zinc comportant quatre chaînes polypeptidiques de 351 amino-acides. Chaque monomère est constitué de deux domaines. Le premier domaine (résidus154-294) permet la fixation du cofacteur tandis que le second intègre le domaine catalytique (résidus 1-153 et 295-351) (Korkhin et al. 1998 ; Goihberg et al. 2010). Cb_s-Adh est proche de son homologue issu de la bactérie thermophile *Thermoanaeobacter brockii* (Tb_s-Adh) par une identité séquence de 75%. En termes de propriétés, les deux enzymes diffèrent par leur thermostabilité (Korkin et al. 1999). Le remplacement, dans Cb_s-Adh de Gln100 par Pro100 dans la séquence d'amino-acides, augmente significativement la thermostabilité de Cb_s-Adh (Goihberg et al. 2007) qui est originellement plus faible.

La capacité productive maximale d'isopropanol est relativement faible (2 à 3 g/L) avec les souches sauvages de C. *beijerinckii* (Chen et al., 1986). La demande de brevet US2009/246842 décrit une méthode de production d'isopropanol dans une souche d'*E.coli* transformé par un vecteur d'expression comprenant le gène codant l'acétyl-CoA transférase de *C. acetobutylicum* (*Ca_thl*), les gènes codant l'acétoacétyl-CoA transférase de C. *acetobutylicum* (*Ca_CtfAB*), le gène codant une acétate décarboxylase (*Ca_adc*) de C. *acetobutylicum* et le gène codant l'alcool déshydrogénase secondaire (*Cb_s-adh*) de C. *beijerinckii.* Dans ce vecteur, l'expression de l'alcool déshydrogénase secondaire est sous le contrôle du promoteur $P_{LlacO-1}$ qui est inductible par IPTG, alors que les expressions des gènes *Ca_thl, Ca_CtfAB* et *Ca_adc* sont sous le contrôle du promoteur natif du gène *Ca_thl* de C. *acetobutylicum.* L'inconvénient de cette méthode est qu'il est nécessaire d'utiliser un milieu de production riche, notamment l'extrait de levure (5 $g.L^{-1}$), et un inducteur, tel que l'isopropyl-β-thiogalactopyranoside ou IPTG. Par ailleurs, l'alcool déshydrogénase secondaire est uniquement exprimée lors qu'IPTG est ajouté dans le milieu de culture, ce qui retarde la production de l'isopropanol et limite la productivité de la fermentation.

La demande internationale WO 11/037414 décrit une autre méthode capable de produire l'isopropanol. Cette méthode consiste à utiliser des microorganismes du genre *Clostridium* transformés par un vecteur d'expression comprenant le gène codant l'acétate décarboxylase (*Ca_adc*) de C. *acetobutylicum*, les gènes codant l'acétoacétyl-CoA transférase de C. *acetobutylicum* (*Ca_CtfAB*) et le gène codant l'alcool déshydrogénase secondaire (*Cb_s-adh)* de C. *beijerinckii.* Ce vecteur nécessite deux promoteurs : un promoteur constitutif *Ca_thlp* qui ne contrôle que l'expression de l'alcool déshydrogénase secondaire et un promoteur inductible $Ca_adc_p$ qui contrôle l'expression des protéines Ca_CtfAB et Ca_Adc. Etant donné que les protéines Ca_CtfAB et Ca_Adc ne s'expriment que pendant la phase de solvantogenèse, l'alcool déshydrogénase secondaire n'a pas de substrat (acétone) avant cette phase. De ce fait, l'isopropanol, le produit final de fermentation, n'est produit qu'après 15 heures de culture, ce qui limite la productivité de l'isopropanol par cette méthode de production.

Par conséquent, il existe toujours une nécessité de développer une méthode de production de l'isopropanol avec une meilleure productivité et un meilleur rendement.

L'objectif de la présente invention est de remédier aux inconvénients de l'art antérieur et de proposer une méthode de production de l'isopropanol par fermentation ayant une meilleure productivité et un meilleur rendement.

## Description de l'invention

Les Inventeurs ont découvert qu'il était possible, de manière avantageuse, de produire de l'isopropanol en utilisant des microorganismes recombinants dans lesquels ont été introduits des vecteurs comprenant les gènes codant les enzymes de la voie métabolique menant de l'acétoacétyl-CoA à l'acétone, à savoir CoA transférase sous-unités A et B et acétoacétate décarboxylase, ainsi que le gène codant une alcool déshydrogénase secondaire de C. *beijerinckii* NRRL B593.

Le premier objectif de la présente invention vise à mettre en disposition d'un vecteur d'expression comprenant :

- les acides nucléiques codant les polypeptides formant un complexe polypeptidique ayant une activité enzymatique permettant de convertir l'acétoacétyl-CoA en acéto-acétate,

- au moins un acide nucléique codant un polypeptide ayant une activité enzymatique permettant de convertir l'acétone en isopropanol, et

- éventuellement, au moins un acide nucléique codant un polypeptide ayant une activité enzymatique permettant de convertir l'acétoacétate en acétone,

l'expression desdits acides nucléiques étant contrôlée par un promoteur constitutif unique situé en amont des susdits acides nucléiques.

On entend par « vecteur d'expression » une molécule d'ADN exogène de forme circulaire, qui est capable de réplication autonome et indépendante de l'ADN chromosomique des cellules hôtes, permettant la transcription et la translation des gènes contenus dans ladite molécule d'ADN par le biais des cellules hôtes.

Dans la présente invention, le terme « vecteur » et le terme « plasmide » peuvent être remplacés l'un par l'autre.

On entend par « promoteur » une région de l'ADN où se lie l'ARN polymérase et qui oriente cette enzyme vers le site où commencera la transcription du gène.

Les promoteurs peuvent être constitutifs ou inductibles.

On entend par « promoteur constitutif » un promoteur non régulé permettant une transcription continue du gène associé.

Au contraire d'un promoteur constitutif, un promoteur inductible permet la transcription du gène associé en réponse à la présence d'un composé particulier, par exemple la présence d'IPTG, ou bien à une condition externe définie, par exemple une température élevée.

On entend par «un promoteur... situé en amont des ... acides nucléiques » un promoteur situé avant les extrémités 5' des acides nucléiques.

La conversion de l'acétoacétyl-CoA en acétoacétate est effectuée par l'acétoacétyl-CoA transférase. Dans la plupart des microorganismes, cette enzyme est formée par deux sous-unités codées par deux gènes différents. Ainsi l'acétoacétyl-CoA transférase de *E.coli* (Ato AD) est formée par les unités A et D, et l'acétoacétyl-CoA transférase de C. *acetobutylicum* (Ca_CtfAB) est formée par les unités A et B.

Dans un mode de réalisation particulier, la présente invention concerne un vecteur comprenant :

- les acides nucléiques codant l'acétoacétyl-CoA transférase (*Ca_CtfAB*)., et
- au moins un acide nucléique codant pour le gène de l'alcool déshydrogénase secondaire de C. *beijerinckii* (*Cb s-adh*); et
- éventuellement au moins un acide nucléique codant pour le gène de l'acétoacétate décarboxylase (*Ca_adc*).

L'expression du gène de l'alcool déshydrogénase secondaire de C. *beijerinckii* NRRL B 593 permet d'assurer la conversion de l'acétone en isopropanol et l'excrétion de ce dernier. Une souche recombinante exprimant cette enzyme peut réduire presque la totalité de l'acétone et de l'acétoïne (3-hydroxy-2-butanone) respectivement en isopropanol et 2,3-butanediol. Bien qu'il soit connu que l'enzyme Cb_s-Adh, de la souche C. *beijerinckii* NRRL B 593, puisse réduire l'acétone et la butanone, l'activité de cette enzyme sur l'acétoïne est constatée pour la première fois par les Inventeurs.

Dans un mode de réalisation plus particulier, la présente invention concerne un vecteur comprenant :

- un acide nucléique décrit dans la séquence SEQ ID NO : 1, ou un acide nucléique ayant une identité de séquence d'au moins 85%, particulièrement 90%, notamment 95% avec la séquence SEQ ID NO : 1, et
- un acide nucléique décrit dans la séquence SEQ ID NO : 2 ou un acide nucléique ayant une identité de séquence d'au moins 85%, particulièrement 90%, notamment 95% avec la séquence SEQ ID NO : 2, et
- un acide nucléique décrit dans la séquence SEQ ID NO : 3 ou un acide nucléique ayant une identité de séquence d'au moins 85%, particulièrement 90%, notamment 95% avec la séquence SEQ ID NO : 3, et
- éventuellement un acide nucléique décrit dans la séquence SEQ ID NO : 4 ou un acide nucléique ayant une identité de séquence d'au moins 85%, particulièrement 90%, notamment 95% avec la séquence SEQ ID NO : 4.

L'acide nucléique décrit dans la séquence SEQ ID NO : 1 code la sous-unité A de l'acétoacétyl-CoA transférase de C. *acetobutylicum* ATCC 824 (*Ca_ctfA*).

L'acide nucléique décrit dans la séquence SEQ ID NO : 2 code la sous-unité B de l'acétoacétyl-CoA transférase de C. *acetobutylicum* ATCC 824 (*Ca_ctfB*).

L'acide nucléique décrit dans la séquence SEQ ID NO: 3 code l'alcool déshydrogénase secondaire de C. *beijerinckii* (*Cb_s-adh*).

L'acide nucléique décrit dans la séquence SEQ ID NO: 4 code l'acétoacétate décarboxylase de C. *acetobutylicum* (*Ca_adc*).

Le pourcentage d'identité entre deux séquences peptidiques ou entre deux séquences d'acides nucléiques peut être calculé selon la formule suivante :

$$\frac{\text{le nombre des résidus identiques x 100}}{\text{le nombre des résidus de la séquence la plus courte}}$$

Les combinaisons des susdits acides nucléiques (ou gènes) forment les opérons *"ipa".* L'expression des gènes des susdits opérons *ipa* étant contrôlée par un promoteur constitutif unique situé en amont des susdits acides nucléiques (ou gènes). Un promoteur constitutif, qui contrôle seul l'expression des opérons *ipa,* autorise une expression constitutive de ceux-ci, ce qui permet à une souche recombinante contenant un des vecteurs de l'invention de produire l'isopropanol dès les premières heures de fermentation.

Grâce à l'expression prématurée des gènes qui conduisent à la production d'isopropanol, le mécanisme de solvantogenèse est déclenché plus tôt dans une souche recombinante contenant un vecteur selon l'invention que dans une souche sauvage. Par conséquent, deux phases de fermentation, à savoir, l'acidogenèse et la solvantogenèse, se déroulent en même temps dans une souche recombinante contenant un vecteur selon l'invention, ce qui permet de diminuer d'au moins 20 heures la durée de fermentation.

Le promoteur constitutif utilisé dans la construction du vecteur de la présente invention peut être le promoteur du gène codant la thiolase de C. *acetobutylicum* ATCC 824, décrit dans la séquence SEQ ID NO : 5, ou le promoteur du gène de la phosphotransbutyrylase de C. *acetobutylicum* ATCC 824, décrit dans la séquence SEQ ID NO : 6.

Dans un mode de réalisation particulier, le vecteur d'expression de la présente invention comprenant :

- un acide nucléique décrit dans la séquence SEQ ID NO : 1, ou un acide nucléique ayant une identité de séquence d'au moins 85%, particulièrement 90%, notamment 95% avec la séquence SEQ ID NO : 1, codant la sous-unité A de l'acétoacétyl-CoA transférase de C. *acetobutylicum* et
- un acide nucléique décrit dans la séquence SEQ ID NO : 2 ou un acide nucléique ayant une identité de séquence d'au moins 85%, particulièrement 90%, notamment 95% avec la séquence SEQ ID NO : 2, codant la sous-unité B de l'acétoacétyl-CoA transférase de C. *acetobutylicum*, et
- éventuellement un acide nucléique décrit dans la séquence SEQ ID NO : 4 ou un acide nucléique ayant une identité de séquence d'au moins 85%, particulièrement 90%, notamment 95% avec la séquence SEQ ID NO: 4, codant l'acétoacétate décarboxylase de C. *acetobutylicum*, et
- un acide nucléique décrit dans la séquence SEQ ID NO : 3 ou un acide nucléique ayant une identité de séquence d'au moins 85%, particulièrement 90%, notamment 95% avec la séquence SEQ ID NO : 3, codant l'alcool déshydrogénase secondaire de C. *beijerinckii*,

l'expression desdits acides nucléiques étant contrôlée par un promoteur constitutif unique situé en amont des susdits acides nucléiques, ledit promoteur étant choisi parmi le promoteur décrit dans la séquence SEQ ID NO : 5 (promoteur thl) et le promoteur décrit dans la séquence SEQ ID NO : 6.

Dans un mode de réalisation particulier, le vecteur de la présente invention comprend :

- un acide nucléique décrit dans la séquence SEQ ID NO : 1,
- un acide nucléique décrit dans la séquence SEQ ID NO : 2,
- un acide nucléique décrit dans la séquence SEQ ID NO : 3,
- un acide nucléique décrit dans la séquence SEQ ID NO : 4, et
- un promoteur décrit dans la séquence SEQ ID NO : 5.

Ce vecteur désigné pFC007 contient, dans l'ordre de l'extrémité 5' vers 3', le gène codant l'alcool déshydrogénase secondaire (*Cb_s-adh*) de C. *beijerinckii* NRRL B 593 ainsi que le gène codant l'acétoacétate décarboxylase (*Ca_adc*) et les gènes codant les sous unités A et B de la coenzyme A transferase (*Ca_CtfAB*) de C. *acetobutylicum* ATCC 824. Le vecteur pFC007 permet la surexpression des gènes de l'opéron *"ipa7"* sous le contrôle d'un seul et unique promoteur constitutif, celui du gène de l'acétyl-CoA transferase (*thiolase*, *Ca_thl*) de C. *acetobutylicum* ATCC 824. Par conséquent, la production de leurs enzymes respectives (Cb_s-Adh, Ca_Adc and Ca_CtfAB) commence dès les premières heures de la fermentation (après environ 4 et 10 heures).

Dans un autre mode de réalisation particulier, le vecteur de la présente invention comprend :

- un acide nucléique décrit dans la séquence SEQ ID NO : 1,

- un acide nucléique décrit dans la séquence SEQ ID NO : 2,
- un acide nucléique décrit dans la séquence SEQ ID NO : 3, et
- un promoteur décrit dans la séquence SEQ ID NO : 5.

Ce vecteur désigné pFC006 contient le gène codant l'alcool déshydrogénase secondaire (*Cb_s-adh*) de C. *beijerinckii* NRRL B 593 (DSMZ 6423) ainsi que les gènes codant les sous unités A et B de la coenzyme A transferase (*Ca_CtfAB*) de C. *acetobutylicum* ATCC 824. Ces gènes sont sous le contrôle d'un seul et unique promoteur constitutif du gène de l'acétyl-CoA transferase (ou *thiolase ou Ca_thl*) de C. *acetobutylicum* ATCC 824.

Les vecteurs d'expression selon la présente invention peuvent comprendre en outre tout autre élément nécessaire pour la transcription et la traduction des enzymes susmentionnées, tel qu'une région RBS (ribosome binding site), un ou plusieurs gènes de sélection.

Une région RBS permet la fixation du ribosome à la molécule d'ARN messager avant le codon d'initiation d'un gène. La séquence de région RBS utilisée dans les vecteurs de la présente invention peut être une séquence quelconque de région RBS d'origine procaryote connue de l'homme du métier.

La présence d'un gène de sélection dans un vecteur selon l'invention permet de sélectionner les microorganismes transformés avec succès. Un gène de sélection dans un vecteur permet aux microorganismes comprenant ledit gène de survivre dans certaines conditions particulières, ou bien d'aboutir à la présence d'une molécule repérable facilement. Le gène de sélection utilisé dans les vecteurs de la présente invention peut être tous gènes de sélection connus de l'homme du métier, notamment un gène permettant aux microorganismes de résister un antibiotique, tel que le gène *Amp*, ou *ErmB.*

Les vecteurs d'expression comportent également des origines de réplication, qui permettent l'initiation de la réplication desdits vecteurs dans les microorganismes hôtes. L'origine de réplication utilisée dans les vecteurs de la présente invention peut être une quelconque origine connue de l'homme du métier.

La présente invention vise également à mettre à disposition un microorganisme recombinant pour la production de l'isopropanol avec une meilleure productivité et un meilleur rendement.

Ledit microorganisme comprend un vecteur d'expression tel que décrit dans la présente invention.

Particulièrement, ledit microorganisme comprend un vecteur comprenant :

- un acide nucléique décrit dans la séquence SEQ ID NO : 1, ou un acide nucléique ayant une identité de séquence d'au moins 85%, particulièrement 90%, notamment 95% avec la séquence SEQ ID NO : 1, et
- un acide nucléique décrit dans la séquence SEQ ID NO : 2 ou un acide nucléique ayant une identité de séquence d'au moins 85%, particulièrement 90%, notamment 95% avec la séquence SEQ ID NO : 2, et
- un acide nucléique décrit dans la séquence SEQ ID NO : 3 ou un acide nucléique ayant une identité de séquence d'au moins 85%, particulièrement 90%, notamment 95% avec la séquence SEQ ID NO : 3, et
- éventuellement un acide nucléique décrit dans la séquence SEQ ID NO : 4 ou un acide nucléique ayant une identité de séquence d'au moins 85%, particulièrement 90%, notamment 95% avec la séquence SEQ ID NO : 4,

l'expression desdits acides nucléiques étant contrôlée par un promoteur constitutif unique situé en amont des susdits acides nucléiques, ledit promoteur étant choisi parmi le promoteur décrit dans la séquence SEQ ID NO : 5 (promoteur thl) et le promoteur décrit dans la séquence SEQ ID NO : 6.

Dans un mode de réalisation avantageux, ledit microorganisme comprend le vecteur pFC007.

Dans un autre mode de réalisation avantageux, ledit microorganisme comprend le vecteur pFC006.

Ledit microorganisme de la présente invention peut être choisi parmi les bactéries du genre *Clostridium,* telles que C. *acetobutylicum*, *C. beijerinckii*, C. *saccharoperbutyacetonicum*, *C. saccharobutylicum*, les bactéries du genre *Bacillus,* telles que *Bacillus subtilis,* ou des entérobactéries, telles que *E.coli.*

Dans un mode de réalisation particulier, le microorganisme selon l'invention est C. *acetobutylicum.*

Dans un mode de réalisation plus particulier, le microorganisme selon l'invention est la souche C. *acetobutylicum* ATCC 824.

Dans un mode de réalisation particulièrement avantageux, l'invention concerne une souche recombinante de C. *acetobutylicum* ATCC 824 comprenant le vecteur pFC007.. La souche recombinante est nommée ATCC 824(pFC007).

Dans un autre mode de réalisation particulièrement avantageux, l'invention concerne une souche recombinante de C. *acetobutylicum* ATCC 824 comprenant le vecteur pFC006. La souche recombinante est nommée ATCC 824(pFC006).

Pour les deux souches recombinantes (ATCC 824(pFC007) et ATCC 824(pFC006)), la production d'isopropanol peut être détectée dès les 10 premières heures de culture, alors que la trace de solvants (ABE) n'est pas détectable dans la souche sauvage de C. *acetobutylicum* ATCC 824 ou la souche recombinante de C. *acetobutylicum* ATCC 824 dans laquelle est inséré un vecteur qui ne comprend que le gène codant l'alcool déshydrogénase secondaire (*Cb_s-adh*) de C. *beijerinckii* NRRL B 593.

La présente invention a également pour objectif de mettre à disposition une méthode de production d'isopropanol, et/ou de D et *L 2,3* butanediol, avec une meilleure productivité et un meilleur rendement.
Ladite méthode comprend au moins les étapes suivantes :

- cultiver un microorganisme selon l'invention dans un milieu de culture comprenant au moins une source de carbone et une source d'azote, dans des conditions permettant la production d'isopropanol, par exemple le milieu de culture décrit par Gapes et al. (Gapes, 1996)
- récupérer l'isopropanol, et/ou de D et *L 2,3* butanediol, du milieu de culture.

Les milieux de culture sont connus de l'homme du métier.
La source de carbone peut être l'amidon, le glucose, le xylose, les hydrolysats lignocellulosiques.
La source d'azote peut être l'ammoniaque, l'extrait de levure, la peptone, l'urée.
Les conditions de culture de microorganisme du genre *Clostridium, Bacillus* ou des bactéries *E. coli* sont connues de l'homme du métier.
La récupération de l'isopropanol, et/ou de D et *L 2,3* butanediol, du milieu de culture est mise en oeuvre par distillation (Mujiburohman et al. 2006).
Les exemples et figures présentés ci-après permettent d'illustrer plus en détail la présente invention. Cependant, la portée de la présente d'invention ne doit en aucun cas être limitée à ces exemples ou figures.

## Figures

Figure 1 : cette figure illustre la construction du vecteur pFC007 à partir du plasmide pMTL500E.
Figure 2 : cette figure présente les sites de restriction du plasmide pMTL500E.
Figure 3 : cette figure illustre la carte de restriction du vecteur pFC007.

## Exemples

### Matériels et méthodes

### Matériel

Les kits utilisés lors des travaux de biologie moléculaire (construction des vecteurs plasmidiques) sont présentés dans le Tableau 1.

**Tableau 1 : Kits et matériels utilisés lors des travaux de biologie moléculaire**

| Nom du kit | Utilisation | Fournisseur |
|---|---|---|
| Enzymes de restriction : *apaI*, *speI*, *sphI*, *xbaI* et *xhoI* | Digestion enzymatiques des ADN plasmidiques | New England Biolabs (NEB) |
| High Fidelity PCR Master Mix™ | Amplification PCR | Roche |
| DreamTaq™ | Amplification PCR | Fermentas |
| Oligonucléotides (Tableau 3) | Amorces pour les réactions de PCR, créées par les inventeurs | BaseClear |
| Alkaline phosphatase | déphosphorylation des extrémités 5' des vecteurs linéarisés empêchant l'autoligation ligation de fragment d'ADN | New England Biolabs |
| T4 DNA ligase | | New England Biolabs (NEB) |
| GenJETTM plasmid miniprep | Extraction de l'ADN plasmidique à partir de cultures *d' E. coli* | Fermentas |
| GenElute™ Bacterial Genomic DNA | Extraction de l'ADN génomique de bactéries (gram positif et négatif) | Sigma-Aldrich |

(suite)

| Nom du kit | Utilisation | Fournisseur |
|---|---|---|
| Z-Competent™ *E. coli* transformation | Production de bactéries *E.coli* XL1 blue et *E. coli* BW25113 chimiocompétentes | Zymo Research |

Les souches utilisées dans les exemples de la présente invention sont :

- *C. acetobutylicum* ATCC 824, provenant de la collection ATCC
- *C. beijerinckii* NRRL B 593, provenant de la collection DSMZ (numéro 6423)
- *E.coli* XL1 bleu, utilisée pour le clonage et le maintien des plasmides
- *E.coli* DH10B (PAN2), utilisée pour la méthylation des plasmides

**Dosage des solvants (acétone, acétoïne, isopropanol, butanol, butanediol) et acides carboxyliques (acides butyrique et acétique)**

Les échantillons de fermentation (2 mL) sont centrifugés 5 minutes à 20 000 g et les surnageants sont utilisés pour le dosage des métabolites de fermentations (glucose, acide acétique et butyrique, 2,3-butanediol, acétone, éthanol, isopropanol et butanol). Pour chaque échantillon, un equivolume d'une solution acid (0.5 mM $H_2SO_4$) contenant l'étalon interne (30 mM de 4-methylvaleric acid) est ajoutée, le mélange est ensuite homogénéisé et filtré (0,2 $\mu$m, Whatman). 10 $\mu$L d'échantillon sont ensuite injectés et analysés dans une colonne Shodex lonpack KC-811 (RP). Les composés dosés sont détectés en sortie de colonne grâce à deux détecteurs : un refractomètre (Waters 2487) et un spectrophotomètre (Waters 2487) mesurant l'absorbance à 210 nm. L'éluant utilisé est une solution d'acide sulfurique à 3 mM, le débit de l'éluant est de 1 mL/min et la température de la colonne est fixée 85 °C.

**Construction des vecteurs**

Les structures génétiques des vecteurs utilisés ou construits dans les exemples de la présente invention sont résumées dans le Tableau 2 ci-après.

**Tableau 2 : Structures génétiques des vecteurs**

| Plasmide | Clostridium/E. coli marqueur sélectif | Description | Référence |
|---|---|---|---|
| pMTL500E | *ermB*/*amp*$^R$ | *Vecteur vide* | Oultram et al., 1988 |
| pFC002 | *ermB*/*amp*$^R$ | *Ca_thl*$_p$ *[Cb-s-adh]* | Ce document |
| pFC005 | *ermB*/*amp*$^R$ | *Ca_thl*$_p$ *[Cb_s-adh; Ca_adc]* | Ce document |
| pFC006 | *ermB*/*amp*$^R$ | *Ca_thl*$_p$ *[Cb_s-adh; Ca_ctfAB]* | Ce document |
| pFC007 | *ermB*/*amp*$^R$ | *Ca_thl*$_p$ *[Cb_s-adh; Ca_adc; Ca_ ctfAB]* | Ce document |
| pAN2 | *ttc* | | (Mermelstein, 1993; Heap, 2009) |

*ermB : gène de résistance à l'érythromycine ; amp*$^R$ *: gène de résistance à l'ampicilline; ttc : gène de résistance à la tetracycline; Ca : ce préfixe indique que le gène provient de* C. acetobutylicum ATCC 824 ; *Cb :* ce préfixe indique que le gène provient de C. *beijerinckii NRRL B593* ; *Ca_thl* : gène de la thiolase ; *Cb_s-adh* : gène de la déshydrogénase secondaire de C. *beijerinckii* NRRL B593; *Ca_adc* : gène de l'acétate décarboxylase de C. *acetobutylicum* ATCC 824 ; *Ca_CtfAB* : gène de la CoA transférase de C. *acetobutylicum* ATCC 824.

La construction du vecteur pFC007 à partir du plasmide pMTL500E (figure 2) nécessite au moins 3 étapes (figure 1).

Etape 1 : Construction du vecteur pFC002

Le vecteur pFC002 est construit à partir du plasmide pMTL500E. Ce dernier est digéré par les enzymes de restriction *sphI* et *xhoI* sur le gène *lacZ.* Le produit de digestion est ensuite déphosporylé. Les sites de restriction *sphI* et *apaI* sont ajoutés respectivement en 5' et 3' de la séquence d'ADN du promoteur *PthI,* par la technique PCR en utilisant les amorces *Ca_thlp-for* et *Ca_thlp_rev* provenant de l'ADN génomique de C. *acetobutyliccum* ATCC 824 (Nolling, J., et al., 2001) (NCBI référence : NC_003030.1 (http://www.ncbi.nlm.nih.gov/nuccore/NC 003030) (Tableau 3). Les sites de restriction *apaI* et *xhoI* sont ajoutés respectivement en 5' et 3' de la séquence d'ADN du gène *Cb_s-adh* de C. *beijerinckii* NRRL B593, par la technique PCR en utilisant les amorces *Cb_s-adh-for* et *Cb_s-adh-rev* (Tableau 3).

Le produit de PCR ainsi obtenu est digéré par les enzymes de restriction *apaI* et *xhoI.* Le plasmide digéré, le promoteur digéré et le gène digéré issus de la PCR sont ligaturés ensemble pour donner un vecteur appelé pFC002 (figure 2). Ce vecteur est maintenu et multiplié dans la souche *E coli* XL1 blue.

Etape 2 : Construction du vecteur pFC006 à partir du plasmide pFC002

Le vecteur pFC002 est digéré par les enzymes de restriction *xhoI* et *XbaI.* Les gènes codant Ca_CtfAB sont amplifiés ensemble à partir de l'ADN génomique de C. *acetobutylicum* ATCC 824, en utilisant les amorces *Ca_CtfAB for* et *Ca_CtfAB rev* (Tableau 3). Le produit de PCR est digéré par les enzymes de restriction *xhoI* et *speI.* Le vecteur pFC002 digéré et le fragment d'ADN digéré contenant le gène *Ca_CtfAB* sont ligaturés ensemble. Le vecteur ainsi obtenu est désigné pFC006 (Figure 1). Le vecteur est maintenu et multiplié dans la souche *E.coli* XL1 blue.

Etape 3 : Construction du vecteur pFC007 à partir du plasmide pFC006

Le vecteur pFC006 est digéré par les enzymes de restriction *xhoI* et *XbaI.* Le gène codant Ca_Adc est amplifié à partir de l'ADN génomique de C. *acetobutylicum* ATCC 824, en utilisant les amorces *Ca_adc for* et *Ca_adc rev* (Tableau 3). Le produit de PCR est digéré par les enzymes *xhoI* et *SpeI.* Le vecteur pFC006 digéré et le fragment d'ADN digéré contenant le gène *Ca_adc* sont ligaturés ensemble. Le vecteur ainsi obtenu est désigné pFC007 (Figures 1 et 3). Le vecteur est maintenu et multiplié dans la souche *E.coli* XL1 blue.

**Tableau 3 : Amorces utilisées pour la construction des opérons *ipa***

| Amorces | Séquence (5'-3') |
|---|---|
| *Ca_thlp-for* | **GCATGC**GAATTTAGAATGAAGTTTCTTATGCA (SEQ ID NO : 7) |
| *Ca_thlp_rev* | **GGGCCC**CCATAGTTTATCCCTAATTTATACG (SEQ ID NO : 8) |
| *Cb_s-adh-for* | **GGGCCC**TTAGACTATTAAAGGAATATTTTTAAGG (SEQ ID NO : 9) |
| *Cb_s-adh-rev* | TTTT**CTCGAG**GTATAATCCTCCATGATCTATTATG (SEQ ID NO: 10) |
| *Ca_ctf A &B for* | CAACTA**CTCGAG**ATAATTTT**TCTAGA**GAATTTAAAA**GGAGG**GATTAAAATG (SEQ ID NO : 11) |
| *Ca_ctf A &B rev* | AATGGT**ACTAGT**TATTTTTT**GTCGAC**TGTTTCATAGTATTTCTTTCTAAACAGCC (SEQ ID NO : 12) |
| *Ca_adc for* | AAACAA**CTCGAG**TTATAA**TCTAGA**TATAAATAAATAGGACTA**GAGG**CG (SEQ ID NO : 13) |
| *Ca_adc rev* | AAAAAT**ACTAGT**TACCATTTAA**GTCGAC**TCTTATTTTTATTACTTAAG (SEQ ID NO : 14) |

Les sites de restriction ajoutés (*sphI*, *apaI, xhoI*, *SalI, xbaI and speI*) sont soulignés. Les sites de fixation des ribosomes sont indiqués en gras.
Le préfixe "Ca" signifie que l'amorce est utilisée sur de l'ADN génomique de C. *acetbutylicum*
Le préfixe Cb signifie que l'amorce est utilisée sur de l'ADN génomique C. *beijerinckii* NRRL B 593

Par des méthodes similaires, le gène codant Ca_Adc peut être inséré dans le vecteur pFC002 pour obtenir le vecteur pFC005 .

Selon des méthodes similaires, les gènes codant Ca_CtfAB peuvent être ensuite insérés dans le vecteur pFC005 pour obtenir le vecteur pFC008.

**Transformation de *C. acetobutylicum***

Les vecteurs pFC002, pFC006 et pFC007 sont d'abord méthylés respectivement par cotransformation avec le plasmide pAN1 dans la souche *E.coli* DH10B.

La souche sauvage de C. *acetobutylicum* ATCC 824 est ensuite transformée respectivement par les vecteurs pFC002, pFC006 et pFC007 méthylés selon le protocole décrit par Oultram (1988). Les souches de C. *acetobutylicum* ATCC 824 transformées par pFC002, pFC006 et pFC007 sont nommées ATCC 824(pFC002), ATCC 824(pFC006) et ATCC 824(pFC007), respectivement.

**Caractérisation génétique des souches recombinantes**

Afin de confirmer la présence de l'un des vecteurs décrits ci-dessus dans la souche C. *acetobutylicum* ATCC 824 après la transformation, une PCR spécifique au plasmide est effectuée avec différent couple d'amorces. Les résultats montrés dans le Tableau 4 confirment la présence de vecteurs dans les souches recombinantes analysées.

**Tableau 4 : Identification des vecteurs pFC005, pFC006 ou pFC007 dans *C. acetobutylicum* ATCC 824 par PCR**

| PCR mix | Taille de fragments PCR attendues [kbp] pour les couples d'amorces : | | | | |
|---|---|---|---|---|---|
| | Cb_thl$_p$ for Cb_ s-adh rev | Cb_s-adh for Ca_ adc rev | Cb_s-adh for Ca_ctfAB rev | Ca_adc for Ca_ ctfAB rev | Ca_ctfAB for Ca_ adc rev |
| pFC002 | 1,4 | | | | |
| pFC005 | 1,4 | 2,0 | No | No | No |
| pFC006 | 1,4 | No | 2,5 | No | No |
| pFC007 | 1,4 | **2,0** | **3,4** | **2,3** | **No** |
| pFC008 | 1,4 | 3,4 | 2,5 | No | 2,3 |

**Fermentation des souches recombinantes**

Des fermentations ont été réalisées dans un milieu de culture décrit par Gapes et al. (GAPES et al., 1996), contenant 90g/L de glucose avec des souches sauvages, *C. beijerinckii* NRRL B 593 et C. *acetobutylicum* ATCC 824, et les souches recombinantes à savoir C. *acetobutylicum* ATCC 824 transformé respectivement par les vecteurs pFC006 et pFC007. Les résultats de fermentation sont présentés dans le Tableau 5.

**Tableau 5: Performances finales sur glucose par des souches sauvages et recombinantes de *C. beijerinckii* NRRL B 593 et de *C. acetobutylicum* ATCC 824**

| Caractéristique | **NRRL B 593 type sauvage** | **ATCC 824 type sauvage** | **ATCC 824 (pFC002)** | **ATCC 824 (pFC006)** | **ATCC 824 (pFC007)** |
|---|---|---|---|---|---|
| **Glucose consommé [g/L]** | 34,30 | 61,99 | 54,82 | 69,26 | **67,79** |
| **Acide lactique [g/L]** | 0,55 | 0,00 | -0,16 | 0,17 | 0,19 |
| **Acide acétique [g/L]** | **0,83** | 2,86 | 4.61 | 3,24 | **1,61** |
| **Acetoïne [g/L]** | 0,00 | 0,59 | 0,10 | 0,06 | 0,05 |
| **2,3-Butanediol [g/L]** | 0,00 | 0,08 | 0,48 | 0,91 | 0,88 |
| **Acide butyrique [g/L]** | 0,21 | **2,02** | 2.51 | 1,08 | 1,06 |
| **Acétone [g/L]** | 0,17 | 5,70 | 0.05 | 0,35 | 0,09 |
| **Ethanol [g/L]** | 0,12 | 1,26 | 0.31 | 1,34 | 1,71 |
| **Isopropanol [g/L]** | 4,47 | 0,10 | 4.25 | 7,27 | **8,37** |
| **Butanol [g/L]** | 8,08 | 8,98 | 8.22 | 11,80 | **12,95** |
| **IBE ou ABE [g/L]** | 12, 83 | 16,04 | 12.82 | 20,76 | **23,12** |
| **Durée de fermentation [h]*** | 33,5 | 49,5 | >45 | **29,9** | **28,0** |

(suite)

| Caractéristique | **NRRL B 593 type sauvage** | **ATCC 824 type sauvage** | **ATCC 824 (pFC002)** | **ATCC 824 (pFC006)** | **ATCC 824 (pFC007)** |
|---|---|---|---|---|---|
| **Productivité maximale en IBE ou ABE [g/L.h]** | 0,41 | 0,41 | 0.33 | **0,69** | **0,81** |
| **Rendement en IBE ou ABE [g/g substrat.]** | 0,37 | 0,26 | 0.24 | 0,30 | 0,34 |
| **Sélectivité en isopropanol [g/g solvants]** | 0,35 | 0,01 | 0.33 | 0,35 | 0,36 |
| **Sélectivité en acétone [g/g solvants]** | 0,01 | 0,36 | <0.01 | 0,02 | 0,00 |

* temps nécessaire pour atteindre 95% de la production finale de solvant.

**Fermentation du glucose par la souche sauvage *C. acetobutylicum* ATCC 824**

Il faut environ 50 heures de fermentation, pour atteindre 95% de la production finale de solvants. La présence de solvant (butanol) ne peut être détectée qu'après 11 heures d'inoculation. Ni l'isopropanol ni 2,3-butanediol ne sont produits par la souche sauvage C. *acetobutylicum* ATCC 824. La production d'acétone et celle d'acétoïne atteignent respectivement 5,7 g/L et 0,6 g/L. Les productions finales en butanol et en éthanol atteignent respectivement 9,0 g/L et 1,3 g/L.

**Fermentation du glucose par la souche recombinante *C. acetobutylicum* ATCC 824(pFC002)**

*C. acetobutylicum* ATCC 824(pFC002) est une souche recombinante ayant reçu le vecteur pFC002. Le vecteur pFC002 permet, à son hôte, l'expression constitutive du gène de l'alcool déshydrogenase secondaire de C. *beijerinckii* NRRL B 593 (*Cb_s-adh*) codant pour l'enzyme Cb_s-Adh. La production de Cb_s-Adh dans la souche recombinante ATCC 824(pFC002) permet d'hydrogéner l'acétone et l'acétoïne respectivement en isopropanol et 2,3-butanediol. L'acétoïne est produite naturellement par la souche sauvage de C. *acetobutylicum* ATCC 824. Cependant, cette souche recombinante n'est pas capable de produire plus d'isopropanol que la souche sauvage de C. *beijerinckii* NRRL B593. De plus, la souche ATCC 824(pFC002) présente des performances de fermentations médiocres, ainsi la production d'isopropanol, et de solvants en générale, de la souche ATCC 824(pFC002) est inférieure de 25% à la production d'acétone et de 20 % à la production de solvants de la souche sauvage de *C. acetobutylicum* ATCC 824. L'acétate et le butyrate restent des produits majoritaires en fin de la fermentation. La production importante d'acides provoque la chute du rendement en solvants, puis qu'une partie des glucides est utilisée pour la production d'acides.

**Fermentation du glucose par la souche recombinante *C. acetobutylicum* ATCC 824(pFC006)**

Comme la souche recombinante ATCC 824(pFC002), la souche recombinante ATCC 824(pFC006) produit de l'isopropanol et du 2,3-butanediol au lieu d'acétone et d'acétoïne.

Cependant, au contraire du profil de production pour la souche recombinante ATCC 824(pFC002), la souche recombinante ATCC 824(pFC006) peut atteindre un niveau élevé de production de solvants sur une durée de temps plus courte. Le temps nécessaire pour atteindre 95% de la production finale de solvant pour cette souche est d'environ 30 heures. La consommation de l'acide butyrique est plus efficace que celle de la souche sauvage ou de la souche recombinante ATCC 824(pFC002), mais reste incomplète. La production de solvants dans cette souche commence plus tôt que pour la souche sauvage ou pour la souche recombinante ATCC 824(pFC002). La production d'isopropanol peut être détectée dès les 10 premières heures de culture de la souche ATCC 824(pFC006), alors que des traces de solvants ne sont pas encore détectables dans la souche sauvage ou dans la souche ATCC 824(pFC002).

De plus, l'incorporation de l'acétate et celle du butyrate dans la souche ATCC 824(pFC006) sont toutes deux améliorées par rapport à celles dans la souche sauvage, ce qui permet de d'avoir un rendement pour l'isopropanol meilleur que le rendement en acétone de la souche sauvage. Pour la souche ATCC 824(pFC007), les titres respectifs de l'isopropanol, du butanol et de l'éthanol est de 7,3 g/L, 11,8 g/L et 1,37 g/L, autrement dit 28 %, 31 % et 6 % supérieur à ceux de la souche sauvage. La productivité est augmentée de 0,41 g/L.h pour une souche sauvage à 0,69 g/L.h pour la souche ATCC 824(pFC007).

La souche ATCC 824(pFC006) produit deux fois plus de 2,3-butanediol que la souche ATCC 824(pFC002), cette augmentation de la production de 2,3-butanediol peut être due à un flux métabolique globalement amélioré.

Il s'avère que l'amélioration de l'incorporation d'acides permet à la souche ATCC 824(pFC006) de produire moins d'acides et d'augmenter sa production de solvants.

Cependant, malgré la surexpression de l'enzyme Ca_CtfAB, la production finale d'acétate est plus élevée dans la souche ATCC 824(pFC006) que dans la souche sauvage, ce qui peut être dû à la production élevée d'acides du fait d'un flux métabolique globalement amélioré.

**Fermentation du glucose par la souche recombinante *C. acetobutylicum* ATCC 824 transformé par pFC007**

La souche ATCC 824(pFC007) présente un profil de fermentation similaire à celui de la souche ATCC 824(pFC006). Le temps pour atteindre 95 % de la production finale de solvants est de moins de 30 heures. L'acidogénèse commence normalement. La production de solvants dans la souche ATCC 824(pFC007) commence pendant la phase exponentielle et plus tôt que dans la souche sauvage. La présence d'isopropanol peut être détectée dans le milieu de culture de la souche ATCC 824(pFC007), 4 heures seulement après l'inoculation alors que l'acétone n'est pas encore détectable dans le milieu de culture d'une souche sauvage.

De plus, l'incorporation de l'acétate et celle du butyrate dans la souche ATCC 824(pFC007) sont toutes deux améliorées par rapport à celles dans la souche ATCC 824(pFC006), ce qui permet de d'avoir un meilleur rendement pour l'isopropanol par rapport à celui de la souche ATCC 824(pFC006), ou d'autres souches décrites précédemment. Dans la souche ATCC 824(pFC007), les titres respectifs de l'isopropanol, du butanol et de l'éthanol est de 8,4 g/L, 13,0 g/L et 1,71 g/L, autrement dit 46 %, 44 % et 36 % supérieurs à ceux de la souche sauvage. La productivité est augmentée de 0,41 g/L.h pour une souche sauvage à 0,81 g/L.h pour la souche ATCC 824(pFC007).

La production de 2,3-butanediol, de la souche ATCC 824(pFC007) est similaire à celle de la souche ATCC 824(pFC006). Comme pour la souche ATCC 824(pFC006), cette augmentation de la production de 2,3-butanediol peut être dû à un flux métabolique globalement amélioré.

**Fermentation du glucose par la souche sauvage *C. beijerinckii* NRRL B 593**

La souche sauvage C. *beijerinckii* NRRL B 593 est cultivée dans les mêmes conditions que la souche C. *acetobutylicum* ATCC 824 sauvage ou recombinante.

Après 34 heures de fermentation, la souche sauvage de C. *beijerinckii* atteint 95% de la production finale de solvants. En revanche, les productions finales de cette souche ne sont que 4,5 g/L pour l'isopropanol, 8,1 g/L pour le butanol et 0,1 g/L pour l'éthanol, ce qui est plus faible que pour la souche sauvage C. *acetobutylicum* ATCC 824 et pour les souches C. *acetobutylicum* ATCC 824 recombinantes (souches ATCC 824(pFC006) et ATCC 824(pFC007).

Comme attendu, la souche C. *beijerinckii* NRRL B 593 sauvage ne produit ni l'acétoïne ni butanediol.

**Conclusion**

Les souches recombinantes ATCC 824(pFC006) et ATCC 824(pFC007) présentent toutes deux une meilleure productivité en solvants que la souche sauvage C. *acetobutylicum* ATCC 824 ou que la souche sauvage C. *beijerinckii* NRRL B 593. La productivité de solvants est multipliée respectivement par un facteur 1,8 avec la souche ATCC 824(pFC006) et par facteur 2 avec la souche ATCC 824(pFC007). Ces résultats montrent que ces deux souches peuvent produire plus de solvants sur une durée de temps plus courte que les souches sauvages. De plus, la souche ATCC 824(pFC007) a montré une bonne aptitude à la culture en continue mono-étagé (une seul fermenteur). Les productions d'isopropanol, butanol et éthanol observées étaient à celles connues pour la souche C. *beijerinckii* NRRL B 593 (Shrikant A., 2011) et C. *acetobutylicum* ATCC824(pFC007) (Godin et al., 1990)

**Référence Bibliographiques**

Chen JS (1995) Alcohol-Dehydrogenase - Multiplicity and Relatedness in the Solvant-Producing Clostridia. FEMS Microbiol Rev 17:263-273

Chen J-S, Hiu SF (1986) Acetone-butanol-isopropanol production by Clostridiumbeijerinckii (synonym,Clostridium butylicum). Biotechnol Lett 8:371-376

Gapes JR, Nimcevic D, Friedl A (1996) Long-Term Continuous Cultivation of Clostridium beijerinckii in a Two-Stage Chemostat with On-Line Solvent Removal. Appl Environ Microbiol 62:3210-3219
George HA, Johnson JL, Moore WEC, Holdeman LV, Chen JS (1983) Acetone, Isopropanol, and Butanol Production by Clostridium beijerinckii (syn. Clostridium butylicum) and Clostridium aurantibutyricum. Appl. Environ. Microbiol. 45:1160-1163
Goihberg, E., et al., Biochemical and Structural Properties of Chimeras Constructed by Exchange of Cofactor-Binding Domains in Alcohol Dehydrogenases from Thermophilic and Mesophilic Microorganisms. Biochemistry, 2010. 49(9): p. 1943-1953
Hanai T, Atsumi S, Liao JC (2007) Engineered synthetic pathway for isopropanol production in Escherichia coli. Appl Environ Microbiol 73:7814-7818
Heap JT, Pennington OJ, Cartman ST, Carter GP, Minton NP (2007) The ClosTron: A universal gene knock-out system for the genus Clostridium. J Microbiol Methods 70:452-464
Ismaiel AA, Zhu CX, Colby GD, Chen JS (1993) Purification and characterization of a primary-secondary alcohol dehydrogenase from two strains of Clostridium beijerinckii. J Bacteriol 175:5097-5105
Johnson JL, Toth J, Santiwatanakul S, Chen JS (1997) Cultures of "Clostridium acetobutylicum" from various collections comprise Clostridium acetobutylicum, Clostridium beijerinckii, and two other distinct types based on DNA-DNA reassociation. International Journal of Systematic Bacteriology 47:420-424
Jojima T, Inui M, Yukawa H (2008) Production of isopropanol by metabolically engineered Escherichia coli. Applied Microbiol Biotechnol 77:1219-1224
Jones, D.T. and D.R. Woods, Acetone butanol fermentation revisited Microbiological Reviews, 1986. 50(4): p. 484-524
Keis S, Shaheen R, Jones DT (2001) Emended descriptions of Clostridium acetobutylicum and Clostridium beijerinckii, and descriptions of Clostridium saccharoperbutylacetonicum sp. nov. and Clostridium saccharobutylicum sp. nov. Int J Syst Evol Microbiol 51:2095-2103
Korkhin Y, Kalb AJ, Peretz M, Bogin O, Burstein Y, Frolow E (1999) Oligomeric integrity - The structural key to thermal stability in bacterial alcohol dehydrogenases. Protein Science 8:1241-1249
Korkhin Y, Kalb AJ, Peretz M, Bogin O, Burstein Y, Frolow F (1998) NADP-dependent bacterial alcohol dehydrogenases: Crystal structure, cofactor-binding and cofactor specificity of the ADHs of Clostridium beijerinckii and Thermoanaerobacter brockii. J Molecular Biol 278:967-981
Langlykke AF, Peterson WH, Fred EB (1937) Reductive Processes of Clostridium butylicum and the Mechanism of Formation of Isopropyl Alcohol. J Bacteriol 34:443-453
Logsdon JE, Loke RA (2001) Isopropyl alcohol. In: Kirk-Othmer Encyclopedia of Chemical Technology. John Wiley & Sons, Inc.
Mermelstein, L.D., et al., Metabolic engineering of Clostridium acetobutylicum ATCC 824 for increased solvent production by enhancement of acetone formation enzyme activities using a synthetic acetone operon. Biotechnology and Bioengineering, 1993. 42(9): p. 1053-1060
Mermelstein, L.D. and E.T. Papoutsakis, Invivo Methylation in Escherichia-Coli by the Bacillus-Subtilis Phage-Phi-3t-I Methyltransferase to Protect Plasmids from Restriction Upon Transformation of Clostridium-Acetobutylicum Atcc-824. Applied and Environmental Microbiology, 1993. 59(4): p. 1077-1081
Mujiburohman M, Sediawan WB, Sulistyo H (2006) A preliminary study: Distillation of isopropanol-water mixture using fixed adsorptive distillation method. Séparation and Purification Technology 48:85-92
Nolling, J., et al., Genome sequence and comparative analysis of the solvent-producing bacterium Clostridium acetobutylicum. Journal of Bacteriology, 2001. 183(16): p. 4823-4838Osburn OL, Brown RW, Werkman CH (1937) The butyl alcohol-isopropyl alcohol fermentation. J Biol Chem 121:685-695
Oultram JD, Peck H, Brehm JK, Thompson DE, Swinfield TJ, Minton NP (1988) Introduction of Genes for Leucine Biosynthesis from Clostridium-Pasteurianum into Clostridium-Acetobutylicum by Cointegrate Conjugal Transfer. Mol Gen Genetics 214:177-179
Papa A (2005) Propanols. In: Ullmann's Encyclopedia of Industrial Chemistry,. Wiley-VCH Verlag GmbH & Co. KGa, Weinheim
Petersen, D.J. and G.N. Bennett, Purification of acetoacetate decarboxylase from Clostridium acetobutylicum ATCC 824 and cloning of the acetoacetate decarboxylase gene in Escherichia coli. Appl Environ Microbiol, 1990. 56(11): p. 3491-8
Peretz M et al. (1997) Molecular cloning, nucleotide sequencing, and expression of genes encoding alcohol dehydrogenases from the thermophile Thermoanaerobacter brockii and the mesophile Clostridium beijerinckii. Anaerobe 3:259-270
Rogers P, Chen J-S, Zidwick M (2006) Organic acid and solvent production. Part III; butanol, acetone, isopropanol; 1,3 and 1,2-propanediol production; and 2,3-butanediol production. In: Dworkin M (ed) The prokaryotes. A handbook on the biology of bacteria. 3rd édition. Springer, New York, USA, pp 672-755
Survase SA, Jurgens G, van Heiningen A, Granström T. (2011) Continuous production of isopropanol and butanol using Clostridium beijerinckii DSM 6423. Appl Microbiol Biotechnol. Sep;91(5):1305-13.
Wiesenborn DP, Rudolph FB, Papoutsakis ET (1988) Thiolase from Clostridium acetobutylicum ATCC 824 and Its Role in the Synthesis of Acids and Solvents. Appl Environ Microbiol 54:2717-2722
Yan RT, Zhu CX, Golemboski C, Chen JS (1988) Expression of Solvent-Forming Enzymes and Onset of Solvent Production in Batch Cultures of Clostridium beijerinckii ("Clostridium butylicum"). Appl Environ Microbiol 54:642-648

## Revendications

**1.** Vecteur d'expression comprenant :

- un acide nucléique décrit dans la séquence SEQ ID NO : 1, ou un acide nucléique ayant une identité de séquence d'au moins 85%, particulièrement 90%, notamment 95% avec la séquence SEQ ID NO : 1, codant la sous-unité A de l'acétoacétyl-CoA transférase de C. *acetobutylicum* et
- un acide nucléique décrit dans la séquence SEQ ID NO : 2 ou un acide nucléique ayant une identité de séquence d'au moins 85%, particulièrement 90%, notamment 95% avec la séquence SEQ ID NO : 2, codant la sous-unité B de l'acétoacétyl-CoA transférase de C. *acetobutylicum*, et
- éventuellement un acide nucléique décrit dans la séquence SEQ ID NO : 4 ou un acide nucléique ayant une identité de séquence d'au moins 85%, particulièrement 90%, notamment 95% avec la séquence SEQ ID NO : 4, codant l'acétoacétate décarboxylase de C. *acetobutylicum*, et
- un acide nucléique décrit dans la séquence SEQ ID NO : 3 ou un acide nucléique ayant une identité de séquence d'au moins 85%, particulièrement 90%, notamment 95% avec la séquence SEQ ID NO : 3, codant l'alcool déshydrogénase secondaire de C. *beijerinckii*, l'expression desdits acides nucléiques étant contrôlée par un promoteur constitutif unique situé en amont des susdits acides nucléiques, ledit promoteur étant choisi parmi le promoteur décrit dans la séquence SEQ ID NO : 5 (promoteur thl) et le promoteur décrit dans la séquence SEQ ID NO : 6.

**2.** Vecteur d'expression selon la revendication 1, **caractérisé en ce que** ledit vecteur comprend :

- un acide nucléique décrit dans la séquence SEQ ID NO : 1,
- un acide nucléique décrit dans la séquence SEQ ID NO : 2,
- un acide nucléique décrit dans la séquence SEQ ID NO : 3,
- un acide nucléique décrit dans la séquence SEQ ID NO : 4, et
- un promoteur décrit dans la séquence SEQ ID NO : 5.

**3.** Vecteur d'expression selon la revendication 1, **caractérisé en ce que** ledit vecteur comprend :

- un acide nucléique décrit dans la séquence SEQ ID NO : 1,
- un acide nucléique décrit dans la séquence SEQ ID NO : 2,
- un acide nucléique décrit dans la séquence SEQ ID NO : 3, et
- un promoteur décrit dans la séquence SEQ ID NO : 5.

**4.** Microorganisme comprenant un vecteur d'expression selon l'une des revendications 1 à 3.

**5.** Microorganisme selon la revendication 4 , **caractérisé en ce que** ledit microorganisme est choisi dans les bactéries du genre *Clostridium, Bacillus,* ou des entérobactéries.

**6.** Microorganisme selon la revendication 5, **caractérisé en ce que** ledit microorganisme est choisi parmi *Clostridium acetobutylicum*, *Clostridium beijerinckii*, *Clostridium saccharoperbutyacetonicum*, *Clostridium saccharobutylicum.*

**7.** Microorganisme selon la revendication 5, **caractérisé en ce que** ledit microorganisme est *Bacillus subtilis.*

**8.** Microorganisme selon la revendication 5, **caractérisé en ce que** ledit microorganisme est *E.coli.*

**9.** Méthode de production d'isopropanol, et/ou de *D* et *L* 2,3 butanediol, **caractérisée en ce que** ladite méthode comprend :

- cultiver un microorganisme selon l'une quelconque des revendications 4 à 8, dans un milieu de culture comprenant au moins une source de carbone et une source d'azote, dans des conditions permettant la production d'isopropanol,
- récupérer l'isopropanol, et/ou de *D* et *L* 2,3 butanediol, du milieu de culture.

**Patentansprüche**

1. Expressionsvektor, umfassend:

    - eine Nukleinsäure, beschrieben in der Sequenz SEQ ID NO: 1 oder eine Nukleinsäure mit einer Sequenzidentität von mindestens 85 %, besonders 90 %, insbesondere 95 % mit der Sequenz SEQ ID NO: 1, die für die Untereinheit A von Acetoacetyl-CoA-Transferase von C. *acetobutylicum* kodiert, und
    - eine Nukleinsäure, beschrieben in der Sequenz SEQ ID NO: 2 oder eine Nukleinsäure mit einer Sequenzidentität von mindestens 85 %, besonders 90 %, insbesondere 95 % mit der Sequenz SEQ ID NO: 2, die für die Untereinheit B von Acetoacetyl-CoA-Transferase von C. *acetobutylicum* kodiert, und
    - gegebenenfalls eine Nukleinsäure, beschrieben in der Sequenz SEQ ID NO: 4 oder eine Nukleinsäure mit einer Sequenzidentität von mindestens 85 %, besonders 90 %, insbesondere 95 % mit der Sequenz SEQ ID NO: 4, die für Acetoacetat-Decarboxylase von C. *acetobutylicum*, kodiert, und
    - eine Nukleinsäure, beschrieben in der Sequenz SEQ ID NO: 3 oder eine Nukleinsäure mit einer Sequenzidentität von mindestens 85 %, besonders 90 %, insbesondere 95 % mit der Sequenz SEQ ID NO: 3, die für die sekundäre Alkoholdehydrogenase von *C. beijerinckii* kodiert, wobei die Expression der Nukleinsäuren von einem einmaligen konstitutiven Promoter gesteuert wird, der stromaufwärts der oben genannten Nukleinsäuren liegt, wobei der Promoter ausgewählt ist aus dem Promoter, beschrieben in der Sequenz SEQ ID NO: 5 (Promoter thl) und dem Promoter, beschrieben in der Sequenz SEQ ID NO: 6.

2. Expressionsvektor nach Anspruch 1, **dadurch gekennzeichnet, dass** der Vektor umfasst:

    - eine Nukleinsäure, beschrieben in der Sequenz SEQ ID NO: 1,
    - eine Nukleinsäure, beschrieben in der Sequenz SEQ ID NO: 2,
    - eine Nukleinsäure, beschrieben in der Sequenz SEQ ID NO: 3,
    - eine Nukleinsäure, beschrieben in der Sequenz SEQ ID NO: 4, und
    - einen Promoter, beschrieben in der Sequenz SEQ ID NO: 5.

3. Expressionsvektor nach Anspruch 1, **dadurch gekennzeichnet, dass** der Vektor umfasst:

    - eine Nukleinsäure, beschrieben in der Sequenz SEQ ID NO: 1,
    - eine Nukleinsäure, beschrieben in der Sequenz SEQ ID NO: 2,
    - eine Nukleinsäure, beschrieben in der Sequenz SEQ ID NO: 3, und
    - einen Promoter, beschrieben in der Sequenz SEQ ID NO: 5.

4. Mikroorganismus, umfassend einen Expressionsvektor nach einem der Ansprüche 1 bis 3.

5. Mikroorganismus nach Anspruch 4, **dadurch gekennzeichnet, dass** der Mikroorganismus ausgewählt ist aus den Bakterien der Gattung *Clostridium*, *Bacillus* oder Enterobakterien.

6. Mikroorganismus nach Anspruch 5, **dadurch gekennzeichnet, dass** der Mikroorganismus ausgewählt ist aus *Clostridium acetobutylicum*, *Clostridium beijerinckii, Clostridium saccharoperbutyacetonicum*, *Clostridium saccharobutylicum.*

7. Mikroorganismus nach Anspruch 5, **dadurch gekennzeichnet, dass** der Mikroorganismus *Bacillus* subtilis ist.

8. Mikroorganismus nach Anspruch 5, **dadurch gekennzeichnet, dass** der Mikroorganismus *E. coli* ist.

9. Verfahren zur Herstellung von Isopropanol und/oder D- und L-2,3-Butandiol, **dadurch gekennzeichnet, dass** das Verfahren umfasst:

    - Kultivieren eines Mikroorganismus nach einem der Ansprüche 4 bis 8 in einem Kulturmedium, umfassend mindestens eine Kohlenstoffquelle und eine Stickstoffquelle, unter Bedingungen, die die Herstellung von Isopropanol ermöglichen,
    - Gewinnen von Isopropanol und/oder von *D*- und *L-2,3*-Butandiol aus dem Kulturmedium.

## Claims

1. Expression vector comprising:

   - a nucleic acid described in the sequence SEQ ID NO: 1, or a nucleic acid having a sequence identity of at least 85%, particularly 90%, in particular 95% with the sequence SEQ ID NO: 1, coding for subunit A of the acetoacetyl-CoA transferase of C. *acetobulylicum* and
   - a nucleic acid described in the sequence SEQ ID NO: 2 or a nucleic acid having a sequence identity of at least 85%, particularly 90%, in particular 95% with the sequence SEQ ID NO: 2, coding for subunit B of the acetoacetyl-CoA transferase of *C. acetobutylicum*, and
   - optionally a nucleic acid described in the sequence SEQ ID NO: 4 or a nucleic acid having a sequence identity of at least 85%, particularly 90%, in particular 95% with the sequence SEQ ID NO: 4, coding for the acetoacetate decarboxylase of *C. acetobutylicum*, and
   - a nucleic acid described in the sequence SEQ ID NO: 3 or a nucleic acid having a sequence identity of at least 85%, particularly 90%, in particular 95% with the sequence SEQ ID NO: 3, coding for the secondary alcohol dehydrogenase of C. *beijerinckii*,

   the expression of said nucleic acids being controlled by a single constitutive promoter located upstream of the abovementioned nucleic acids, said promoter being selected from the promoter described in the sequence SEQ ID NO: 5 (thl promoter) and the promoter described by the sequence SEQ ID NO: 6.

2. Expression vector according to claim 1, **characterized in that** said vector comprises:

   - a nucleic acid described in the sequence SEQ ID NO: 1,
   - a nucleic acid described in the sequence SEQ ID NO: 2,
   - a nucleic acid described in the sequence SEQ ID NO: 3,
   - a nucleic acid described in the sequence SEQ ID NO: 4, and
   - a promoter described in the sequence SEQ ID NO: 5.

3. Expression vector according to claim 1, **characterized in that** said vector comprises:

   - a nucleic acid described in the sequence SEQ ID NO: 1,
   - a nucleic acid described in the sequence SEQ ID NO: 2,
   - a nucleic acid described in the sequence SEQ ID NO: 3, and
   - a promoter described in the sequence SEQ ID NO: 5.

4. Microorganism comprising an expression vector according to one of claims 1 to 3.

5. Microorganism according to claim 4, **characterized in that** said microorganism is selected from the bacteria of the genera *Clostridium*, *Bacillus*, or from enterobacteria.

6. Microorganism according to claim 5, **characterized in that** said microorganism is selected from *Clostridium acetobutylicum*, *Clostridium beijerinckii*, *Clostridium saccharoperbutylacetonicum*, *Clostridium saccharabutylicum.*

7. Microorganism according to claim 5, **characterized in that** said microorganism is *Bacillus subtilis.*

8. Microorganism according to claim 5, **characterized in that** said microorganism is *E. coli.*

9. Method for the production of isopropanol, and/or of D *and L* 2,3-butanediol, **characterized in that** said method comprises:

   - cultivating a microorganism according to any one of claims 4 to 8, in a culture medium comprising at least one carbon source and one nitrogen source , under conditions allowing the production of isopropanol,
   - recovering the isopropanol, and/or *D* and *L* 2,3-butanediol, from the culture medium.

# FIGURE 1

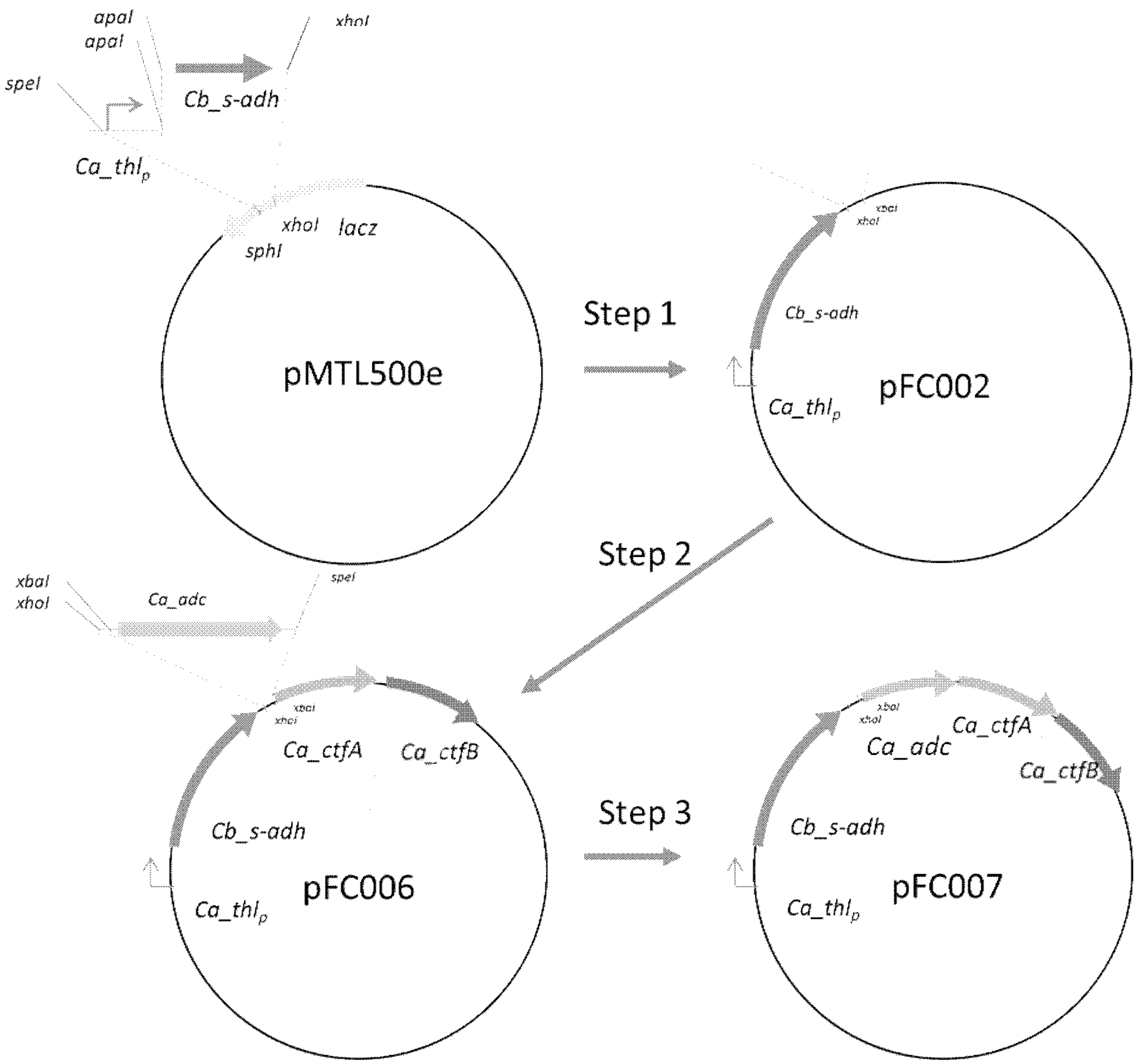
apal
apal
xhol
spel
Cb_s-adh
Ca_thl$_p$
xhol
lacz
sphl
pMTL500e
Step 1
xbal
xhol
Cb_s-adh
Ca_thl$_p$
pFC002
Step 2
xbal
xhol
Ca_adc
spel
xbal
xhol
Ca_ctfA
Ca_ctfB
Cb_s-adh
Ca_thl$_p$
pFC006
Step 3
xbal
xhol
Ca_adc
Ca_ctfA
Ca_ctfB
Cb_s-adh
Ca_thl$_p$
pFC007

FIGURE 2

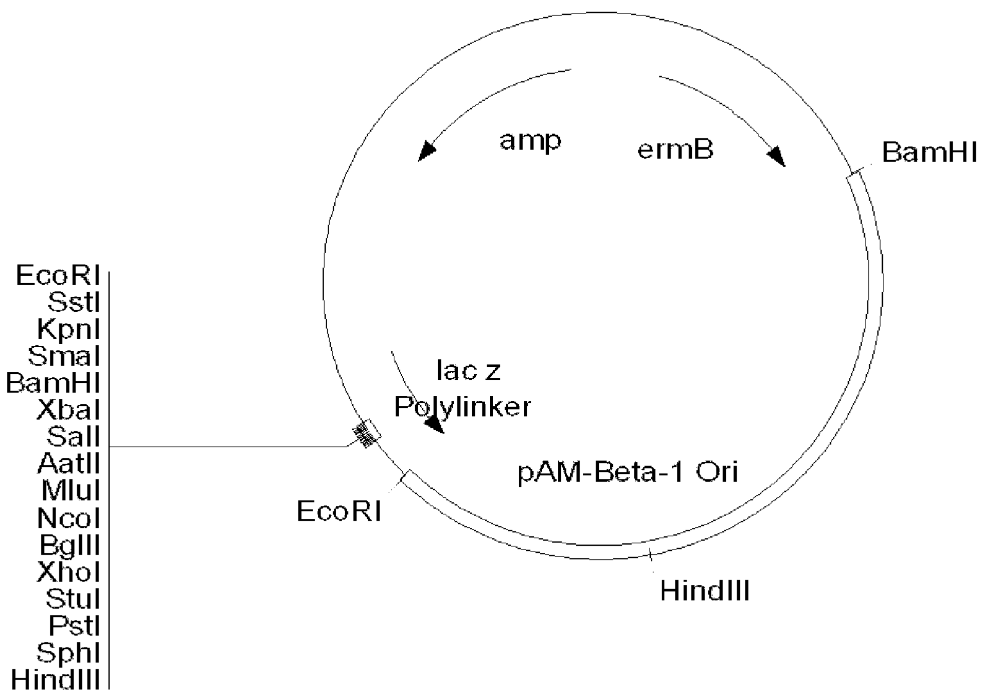
amp
ermB
BamHI
EcoRI
SstI
KpnI
SmaI
BamHI
XbaI
SalI
AatII
MluI
NcoI
BglII
XhoI
StuI
PstI
SphI
HindIII
lac z
Polylinker
pAM-Beta-1 Ori
EcoRI
HindIII

FIGURE 3

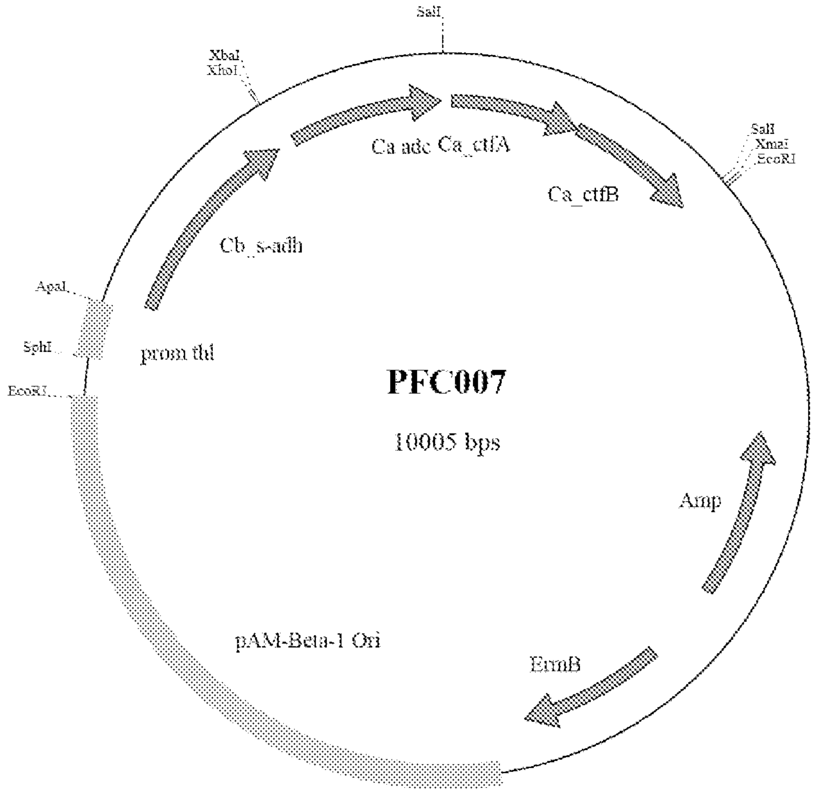
SalI
XbaI
XhoI
SalI
XmaI
EcoRI
Ca adc
Ca_ctfA
Ca_ctfB
Cb_s-adh
ApaI
SphI
EcoRI
prom thl
PFC007
10005 bps
Amp
ErmB
pAM-Beta-1 Ori

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2009246842 A
- WO 11037414 A


### Littérature non-brevet citée dans la description


- **CHEN JS.** Alcohol-Dehydrogenase - Multiplicity and Relatedness in the Solvant-Producing Clostridia. *FEMS Microbiol Rev,* 1995, vol. 17, 263-273
- **CHEN J-S ; HIU SF.** Acetone-butanol-isopropanol production by Clostridiumbeijerinckii (synonym,Clostridium butylicum). *Biotechnol Lett,* 1986, vol. 8, 371-376
- **GAPES JR ; NIMCEVIC D ; FRIEDL A.** Long-Term Continuous Cultivation of Clostridium beijerinckii in a Two-Stage Chemostat with On-Line Solvent Removal. *Appl Environ Microbiol,* 1996, vol. 62, 3210-3219
- **GEORGE HA ; JOHNSON JL ; MOORE WEC ; HOLDEMAN LV ; CHEN JS.** Acetone, Isopropanol, and Butanol Production by Clostridium beijerinckii (syn. Clostridium butylicum) and Clostridium aurantibutyricum. *Appl. Environ. Microbiol.,* 1983, vol. 45, 1160-1163
- **GOIHBERG, E. et al.** Biochemical and Structural Properties of Chimeras Constructed by Exchange of Cofactor-Binding Domains in Alcohol Dehydrogenases from Thermophilic and Mesophilic Microorganisms. *Biochemistry,* 2010, vol. 49 (9), 1943-1953
- **HANAI T ; ATSUMI S ; LIAO JC.** Engineered synthetic pathway for isopropanol production in Escherichia coli. *Appl Environ Microbiol,* 2007, vol. 73, 7814-7818
- **HEAP JT ; PENNINGTON OJ ; CARTMAN ST ; CARTER GP ; MINTON NP.** The ClosTron: A universal gene knock-out system for the genus Clostridium. *J Microbiol Methods,* 2007, vol. 70, 452-464
- **ISMAIEL AA ; ZHU CX ; COLBY GD ; CHEN JS.** Purification and characterization of a primary-secondary alcohol dehydrogenase from two strains of Clostridium beijerinckii. *J Bacteriol,* 1993, vol. 175, 5097-5105
- **JOHNSON JL ; TOTH J ; SANTIWATANAKUL S ; CHEN JS.** Cultures of ''Clostridium acetobutylicum'' from various collections comprise Clostridium acetobutylicum, Clostridium beijerinckii, and two other distinct types based on DNA-DNA reassociation. *International Journal of Systematic Bacteriology,* 1997, vol. 47, 420-424
- **JOJIMA T ; INUI M ; YUKAWA H.** Production of isopropanol by metabolically engineered Escherichia coli. *Applied Microbiol Biotechnol,* 2008, vol. 77, 1219-1224
- **JONES, D.T. ; D.R. WOODS.** Acetone butanol fermentation revisited. *Microbiological Reviews,* 1986, vol. 50 (4), 484-524
- **KEIS S ; SHAHEEN R ; JONES DT.** Emended descriptions of Clostridium acetobutylicum and Clostridium beijerinckii, and descriptions of Clostridium saccharoperbutylacetonicum sp. nov. and Clostridium saccharobutylicum sp. nov. *Int J Syst Evol Microbiol,* 2001, vol. 51, 2095-2103
- **KORKHIN Y ; KALB AJ ; PERETZ M ; BOGIN O ; BURSTEIN Y ; FROLOW E.** Oligomeric integrity - The structural key to thermal stability in bacterial alcohol dehydrogenases. *Protein Science,* 1999, vol. 8, 1241-1249
- **KORKHIN Y ; KALB AJ ; PERETZ M ; BOGIN O ; BURSTEIN Y ; FROLOW F.** NADP-dependent bacterial alcohol dehydrogenases: Crystal structure, cofactor-binding and cofactor specificity of the ADHs of Clostridium beijerinckii and Thermoanaerobacter brockii. *J Molecular Biol,* 1998, vol. 278, 967-981
- **LANGLYKKE AF ; PETERSON WH ; FRED EB.** Reductive Processes of Clostridium butylicum and the Mechanism of Formation of Isopropyl Alcohol. *J Bacteriol,* 1937, vol. 34, 443-453
- Isopropyl alcohol. **LOGSDON JE ; LOKE RA.** Kirk-Othmer Encyclopedia of Chemical Technology. John Wiley & Sons, Inc, 2001
- **MERMELSTEIN, L.D. et al.** Metabolic engineering of Clostridium acetobutylicum ATCC 824 for increased solvent production by enhancement of acetone formation enzyme activities using a synthetic acetone operon. *Biotechnology and Bioengineering,* 1993, vol. 42 (9), 1053-1060

- **MERMELSTEIN, L.D. ; E.T. PAPOUTSAKIS.** Invivo Methylation in Escherichia-Coli by the Bacillus-Subtilis Phage-Phi-3t-I Methyltransferase to Protect Plasmids from Restriction Upon Transformation of Clostridium-Acetobutylicum Atcc-824. *Applied and Environmental Microbiology,* 1993, vol. 59 (4), 1077-1081
- **MUJIBUROHMAN M ; SEDIAWAN WB ; SULISTYO H.** A preliminary study: Distillation of isopropanol-water mixture using fixed adsorptive distillation method. *Séparation and Purification Technology,* 2006, vol. 48, 85-92
- **NOLLING, J. et al.** Genome sequence and comparative analysis of the solvent-producing bacterium Clostridium acetobutylicum. *Journal of Bacteriology,* 2001, vol. 183 (16), 4823-4838
- **OSBURN OL ; BROWN RW ; WERKMAN CH.** The butyl alcohol-isopropyl alcohol fermentation. *J Biol Chem,* 1937, vol. 121, 685-695
- **OULTRAM JD ; PECK H ; BREHM JK ; THOMPSON DE ; SWINFIELD TJ ; MINTON NP.** Introduction of Genes for Leucine Biosynthesis from Clostridium-Pasteurianum into Clostridium-Acetobutylicum by Cointegrate Conjugal Transfer. *Mol Gen Genetics,* 1988, vol. 214, 177-179
- Propanols. **PAPA A.** Ullmann’s Encyclopedia of Industrial Chemistry. Wiley-VCH Verlag GmbH & Co. KGa, 2005
- **PETERSEN, D.J. ; G.N. BENNETT.** Purification of acetoacetate decarboxylase from Clostridium acetobutylicum ATCC 824 and cloning of the acetoacetate decarboxylase gene in Escherichia coli. *Appl Environ Microbiol,* 1990, vol. 56 (11), 3491-8
- **PERETZ M et al.** Molecular cloning, nucleotide sequencing, and expression of genes encoding alcohol dehydrogenases from the thermophile Thermoanaerobacter brockii and the mesophile Clostridium beijerinckii. *Anaerobe,* 1997, vol. 3, 259-270
- Organic acid and solvent production. Part III; butanol, acetone, isopropanol;1,3 and 1,2-propanediol production; and 2,3-butanediol production. **ROGERS P ; CHEN J-S ; ZIDWICK M.** The prokaryotes. A handbook on the biology of bacteria. Springer, 2006, 672-755
- **SURVASE SA ; JURGENS G ; VAN HEININGEN A ; GRANSTRÖM T.** Continuous production of isopropanol and butanol using Clostridium beijerinckii DSM 6423. *Appl Microbiol Biotechnol.,* Septembre 2011, vol. 91 (5), 1305-13
- **WIESENBORN DP ; RUDOLPH FB ; PAPOUTSAKIS ET.** Thiolase from Clostridium acetobutylicum ATCC 824 and Its Role in the Synthesis of Acids and Solvents. *Appl Environ Microbiol,* 1988, vol. 54, 2717-2722
- **YAN RT ; ZHU CX ; GOLEMBOSKI C ; CHEN JS.** Expression of Solvent-Forming Enzymes and Onset of Solvent Production in Batch Cultures of Clostridium beijerinckii (’’Clostridium butylicum’’). *Appl Environ Microbiol,* 1988, vol. 54, 642-648

SEQUENCE LISTING

<110> IFPEN Stichting Dienst Landbouwkundig Onderzoek

<120> PRODUCTION D'ISOPROPANOL PAR DES SOUCHES RECOMBINANTES AMELIOREES

<130> IFB 10 PQ IFP TYLI

<160> 14

<170> PatentIn version 3.5

<210> 1
<211> 646
<212> DNA
<213> Clostridium acetobutylicum

<400> 1

```
atgaactcta aaataattag atttgaaaat ttaaggtcat tctttaaaga tgggatgaca      60
attatgattg gaggtttttt aaactgtggc actccaacca aattaattga tttttttagtt     120
aatttaaata taaagaattt aacgattata agtaatgata catgttatcc taatacaggt     180
attggtaagt taatatcaaa taatcaagta aaaaagctta ttgcttcata tataggcagc     240
aacccagata ctggcaaaaa actttttaat aatgaacttg aagtagagct ctctccccaa     300
ggaactctag tggaaagaat acgtgcaggc ggatctggct taggtggtgt actaactaaa     360
acaggtttag gaactttgat tgaaaaagga aagaaaaaaa tatctataaa tggaacggaa     420
tatttgttag agctacctct tacagccgat gtagcattaa ttaaaggtag tattgtagat     480
gaggccggaa acaccttcta taaaggtact actaaaaact ttaatcccta tatggcaatg     540
gcagctaaaa ccgtaatagt tgaagctgaa aatttagtta gctgtgaaaa actagaaaag     600
gaaaaagcaa tgacccccgg agttcttata aattatatag taaagg                    646
```

<210> 2
<211> 655
<212> DNA
<213> Clostridium acetobutylicum

<400> 2

gcctgcataa aatgattaat gataaaaacc tagcgaaaga aataatagcc aaaagagttg 60

caagagaatt aaaaaatggt caacttgtaa acttaggtgt aggtcttcct accatggttg 120

cagattatat accaaaaaat ttcaaaatta ctttccaatc agaaaacgga atagttggaa 180

tgggcgctag tcctaaaata aatgaggcag ataaagatgt agtaaatgca ggaggagact 240

atacaacagt acttcctgac ggcacatttt tcgatagctc agtttcgttt tcactaatcc 300

gtggtggtca cgtagatgtt actgttttag gggctctcca ggtagatgaa aagggtaata 360

tagccaattg gattgttcct ggaaaaatgc tctctggtat gggtggagct atggatttag 420

taaatggagc taagaaagta ataattgcaa tgagacatac aaataaaggt caacctaaaa 480

ttttaaaaaa atgtacactt cccctcacgg caaagtctca agcaaatcta attgtaacag 540

aacttggagt aattgaggtt attaatgatg gtttacttct cactgaaatt aataaaaaca 600

caaccattga tgaaataagg tctttaactg ctgcagattt actcatatcc aatga 655

<210> 3
<211> 1121
<212> DNA
<213> clostridium beijerinckii

<400> 3

ttagactatt aaaggaatat ttttaaggag gaacatattt tatgaaaggt tttgcaatgc 60
taggtattaa taagttagga tggatcgaaa aagaaaggcc agttgcgggt tcatatgatg 120
ctattgtacg cccattagca gtatctccgt gtacatcaga tatacatact gtttttgagg 180
gagctcttgg agataggaag aatatgattt tagggcatga agctgtaggt gaagttgttg 240
aagtaggaag tgaagtgaag gattttaaac ctggtgacag agttatagtt ccttgtacaa 300
ctccagattg gagatctttg gaagttcaag ctggttttca acagcactca aacggtatgc 360
tcgcaggatg gaaattttca aatttcaagg atggagtttt tggtgaatat tttcatgtaa 420
atgatgcgga tatgaatctt gcgattctac ctaaagacat gccattagaa aatgctgtta 480
tgataacaga tatgatgact actggatttc atggagcaga acttgcagat attcaaatgg 540
gttcaagtgt tgtggtaatt ggcattggag ctgttggctt aatgggaata gcaggtgcta 600
attacgtgga gcaggtagaa taattggagt ggggagcagg ccgatttgtg ttgaggctgc 660
aaaattttat ggagcaacag atattctaaa ttataaaaat ggtcatatag ttgatcaagt 720
tatgaaatta acgaatggaa aaggcgttga ccgcgtaatt atggcaggcg gtggttctga 780
aacattatcc caagcagtat ctatggttaa accaggagga ataatttcta atataaatta 840
tcatggaagt ggagatgctt tactaatacc acgtgtagaa tggggatgtg gaatggctca 900
caagactata aaaggaggtc tttgtcctgg gggacgtttg agagcagaaa tgttaagaga 960
tatggtagta tataatcgtg ttgatctaag taaattagtt acacatgtat atcatggatt 1020
tgatcacata gaagaagcac tgttattaat gaaagacaag ccaaaagact taattaaagc 1080
agtagttata ttataacata atagatcatg gaggattata c 1121

<210> 4
<211> 865
<212> DNA
<213> clostridium acetobutylicum

<400> 4

aaacaattat aatataaata aataggacta gaggcgattt ataatgtgaa gataaagtat 60
gttagaaaag ctaaacatta ttaaatttag gaaggtgact tttatgttaa aggatgaagt 120

aattaaacaa attagcacgc cattaacttc gcctgcattt cctagaggac cctataaatt 180

tcataatcgt gagtatttta acattgtata tcgtacagat atggatgcac ttcgtaaagt 240

tgtgccagag cctttagaaa ttgatgagcc cttagtcagg tttgaaatta tggcaatgca 300

tgatacgagt ggacttggtt gttatacaga aagcggacag gctattcccg taagctttaa 360

tggagttaag ggagattatc ttcatatgat gtatttagat aatgagcctg caattgcagt 420

aggaagggaa ttaagtgcat atcctaaaaa gctcgggtat ccaaagcttt ttgtggattc 480

agatacttta gtaggaactt tagactatgg aaaacttaga gttgcgacag ctacaatggg 540

gtacaaacat aaagccttag atgctaatga agcaaaggat caaatttgtc gccctaatta 600

tatgttgaaa ataataccca attatgatgg aagccctaga atatgtgagc ttataaatgc 660

gaaaatcaca gatgttaccg tacatgaagc ttggacagga ccaactcgac tgcagttatt 720

tgatcacgct atggcgccac ttaatgattt gccagtaaaa gagattgttt ctagctctca 780

cattcttgca gatataatat tgcctagagc tgaagttata tatgattatc ttaagtaata 840

aaaataagat taaatggtaa ttttt 865

<210> 5
<211> 242
<212> DNA
<213> clostridium acetobutylicum

<400> 5

gaatttagaa tgaagtttct tatgcacaag tattttttat tacattaata tagttaaaat 60

ataaacttat gtatttatgc taaaacatga ttttaagggg gttagcatat gcataagttt 120

aatttttttg ttaaaaaata ttaaactttg tgtttttttt aacaaaatat attgataaaa 180

ataataatag tgggtataat taagttgtta gagaaaacgt ataaattagg gataaactat 240

gg 242

<210> 6
<211> 145
<212> DNA
<213> clostridium acetobutylicum

<400> 6

cgactgtgga tggagttaag tcagcagaaa gtataatgag aaaatataaa atataaataa 60

ttttctaaaa aacttaactt catgtgaaaa gtttgttaaa atataaatga gcacgttaat 120

catttaacat agataattaa atagt 145

<210> 7
<211> 32
<212> DNA

<213> artificiel sequence

<400> 7
gcatgcgaat ttagaatgaa gtttcttatg ca 32

<210> 8
<211> 31
<212> DNA
<213> artificiel sequence

<400> 8
gggcccccat agtttatccc taatttatac g 31

<210> 9
<211> 34
<212> DNA
<213> artificiel sequence

<400> 9
gggcccttag actattaaag gaatattttt aagg 34

<210> 10
<211> 35
<212> DNA
<213> artificiel sequence

<400> 10
ttttctcgag gtataatcct ccatgatcta ttatg 35

<210> 11
<211> 55
<212> DNA
<213> artificiel sequence

<400> 11
aatggtacta gttatttttt gtcgactgtt tcatagtatt tctttctaaa cagcc 55

<210> 12
<211> 55
<212> DNA
<213> artificiel sequence

<400> 12
aatggtacta gttatttttt gtcgactgtt tcatagtatt tctttctaaa cagcc 55

<210> 13
<211> 48
<212> DNA
<213> artificiel sequence

<400> 13
aaacaactcg agttataatc tagatataaa taaataggac tagaggcg 48

<210> 14
<211> 48
<212> DNA
<213> artificiel sequence

<400> 14
aaaaatacta gttaccattt aagtcgactc ttatttttat tacttaag 48